## Europäisches Patentamt
## European Patent Office
## Office européen des brevets

(11) Veröffentlichungsnummer: **0 348 535**
**A1**

# EUROPÄISCHE PATENTANMELDUNG

(21) Anmeldenummer: 88108548.4

(22) Anmeldetag: 27.05.88

(51) Int. Cl.4 **A61N 1/36**

(43) Veröffentlichungstag der Anmeldung:
03.01.90 Patentblatt 90/01

(84) Benannte Vertragsstaaten:
**AT BE CH DE ES FR GB GR IT LI LU NL SE**

(71) Anmelder: **Kocsi, István**
**Munkásotthon u. 61**
**H-1043 Budapest(HU)**

Anmelder: **Klauber, András, Dr.**
**Gyakorlo u. 4/f**
**H-1106 Budapest(HU)**

(72) Erfinder: **Kocsi, István**
**Munkásotthon u. 61**
**H-1043 Budapest(HU)**
Erfinder: **Klauber, András, Dr.**
**Gyakorlo u. 4/f**
**H-1106 Budapest(HU)**

(74) Vertreter: **Dipl.-Ing. Schwabe, Dr. Dr.**
**Sandmair, Dr. Marx**
**Stuntzstrasse 16**
**D-8000 München 80(DE)**

(54) Schaltungsanordnung zur funktionsgemässen, elektrischen Stimulierung gelähmter Muskeln oder Muskelgruppen.

(57) Die Erfindung betrifft eine Schaltungsanordnung zur funktionsgemäßen, elektrischen Stimulierung gelähmter Muskeln oder Muskelgruppen mit einem Sensor, der vom Patienten oder einer anderen Person betätigt wird, mit Reizstromquelle und Elektroden zur Übertragung der Reizimpulse.

Bei der erfindungsgemäßen Schaltungsanordnung ist die Reizstromquelle ein von außen getriggerter Burst-Generator, der aus einem, vom Sensor (1) gesteuerten Verzögerungsglied (2) zur Bestimmung der Dauer der Stimulation, einem taktbaren Transverter (3) zur Erzeugung von Stimulationsspannung ($U_S$) durch Vervielfachung der Versorgungsspannung ($U_T$), einem taktbaren Impulsgenerator (5) zur Einstellung der Frequenz und Impulsbreite der stimulierenden Impulse, einem Chopper (6) zur Erzeugung der stimulierenden Impulse aus der Stimulationsspannung ($U_S$), die mit der Frequenz der Impulse vom Impulsgenerator (5) synchronisiert sind, einem Amplitudenregler (7) zur Einstellung der Intensität der stimulierenden Impulse, und einer Versorgungseinheit (11) für die Versorgung der genannten Einheiten mit Betriebsspannung ($U_T$) besteht, wobei der Ausgang des Verzögerungsgliedes (2) zum Takteingang des Transverters (3) und des Impulsgenerators (5), der Ausgang vom Transverter (3) zur Elektrode (9) der einen Polarität, und der Ausgang vom Amplitudenregler (7) zur Elektrode (10) der anderen Polarität geführt sind und der Ausgang des Impulsgenerators (5) an den Steuereingang des Choppers (6) und die Eingänge des Amplitudenreglers (7) an den Ausgang des Transverters (3) und dem ungeerdeten Ende des gesteuerten Kanals des Choppers (6) angeschlossen sind.

Fig 1

## Schaltungsanordnung zur funktionsgemäßen, elektrischen Stimulierung gelähmter Muskeln oder Muskelgruppen

Die Erfindung betrifft eine Schaltungsanordnung zur funktionsgemäßen, elektrischen Stimulierung gelähmter Muskeln oder Muskelgruppen mit einem Sensor, der vom Patienten oder einer anderen Person betätigt wird, mit Reizstromquelle und Elektroden zur Übertragung der Reizimpulse. Mit Hilfe der erfindungsgemäßen Schaltungsanordnung sind Patienten imstande, die ausgefallenen Muskeln oder Muskelgruppen zur normalen Funktion zu bringen. Voraussetzung für eine Stimulierung und dadurch die Wiederherstellung einer Muskelfunktion ist ein intakter Muskel-Nerv-Übergang.

Reizstromgeräte der oben genannten Art sind in vielen Ländern bekannt, aber sie besitzen einen komplizierten Aufbau und sind nur auf einem speziellen Gebiet (z. B. Beinhängen wegen Peroneus-Lähmung, Scoliosis Behandlung, usw.) einsetzbar.

Es ist z. B. ein Apparat zur Behandlung der Peroneus-Lähmung der jugoslawischen Firma ZAVOD SRSZA Rehabilitacijo Invalido mit einem komplizierten Transistorschaltkreis bekannt. Zur Verminderung des unangenehmen Stechgefühls wird eine Impulsreihe mit logarithmisch steigender Amplitude erzeugt, wobei die gewünschte Wirkung jedoch nicht erreicht wird, weil die Muskeln als Komparator wirken.

Bei einem anderen Apparat mit der Bezeichnung ISOMOD der amerikanischen Firma MEDTRONIC werden implantierte Elektroden verwendet und der Apparat funktioniert im Zählersystem, d. h. die Burst-Zeiten und die Frequenz der Impulsreihe werden nicht zeitlich, sondern mit der Anzahl der Impulse in einer Zeiteinheit bestimmt. Diese Methode wird mit einem unnötig komplizierten Stromkreis verwirklicht. Ein weiterer Nachteil ergibt sich daraus, daß der Stromkreis auch in den Burst-Pausen den gleichen Strom aufnimmt, und so die Lebensdauer der Batterien nur 10 - 20 Betriebsstunden beträgt.

Eine Vorrichtung zur Behandlung der weiblichen urinösen Inkontinenz ist aus der DE-AS 19 47 412 bekannt. Bei dieser Vorrichtung wird ein zum Einfügen in die Vagina geformtes Einfügungsstuck mit in Längsrichtung gegeneinander versetzten Elektroden zur Übertragung der Stimulationsimpulse beschrieben, bei der eine einfache Impulsgeberschaltung zur Anwendung kommt. Die vorgeschlagene Impulsgeberschaltung liefert eine Impulsreihe mit einer maximalen Spitzenspannung von 20 V und einer konstanten Frequenz von 25 bis 200 Hz. Zur Änderung der Impulsparameter muß man einen anderen Stromkreis wählen.

In der DE-PS 25 38 369 wird eine Schaltungsanordnung für ein intravaginales Reizstromtherapiegerät für die Korrektur der Inkontinenz von Patienten mit einer Oszillatorschaltung beschrieben, wobei die Oszillatorschaltung ein astabiler Multivibrator ist und die Einstellung der gewünschten Frequenz durch Veränderung eines Widerstandes ermöglicht ist. Auch bei dieser Lösung bleibt die Amplitude der stimulierenden Impulse konstant, wodurch ein Nachlassen der Stimulierfähigkeit der Muskeln entsteht.

In der US-PS 4,406,288 wird eine Schaltungsanordnung zur Blasenstimulierung vorgeschlagen, die einen Impulsgenerator und einen Verstärker, dessen Ausgang über einen Transformator und ein Potentiometer mit den Elektroden verbunden ist, aufweist. Bei dieser Lösung kann das Signal des Verstärkers mit einem DG-Signal beaufschlagt werden. Weiterhin können die Frequenz und die Amplitude voreingestellt werden. Da diese Signalparameter während der Stimulierung konstant bleiben, sind sie für eine effektive Stimulierung nicht geeignet.

Der Erfindung liegt daher die Aufgabe zugrunde, eine Schaltungsanordnung zur funktionsgemäßen, elektrischen Stimulierung gelähmter Muskeln oder Muskelgruppen zu schaffen, die es ermöglicht, von außen getriggerte stimulierende Impulse mit variabler Amplitude, Frequenz und Impulsbreite zu erzeugen.

Erfindungsgemäß wird diese Aufgabe durch eine Schaltungsanordnung gelöst, bei der die Reizstromquelle ein von außen getriggerter Burst-Generator ist, der aus einem, vom Sensor gesteuerten Verzögerungsglied zur Bestimmung der Dauer der Stimulation, einem taktbaren Transverter zur Erzeugung von Stimulationsspannung durch Vervielfachung der Versorgungsspannung, einem taktbaren Impulsgenerator zur Einstellung der Frequenz und Impulsbreite der stimulierenden Impulse, einem Chopper zur Erzeugung der stimulierenden Impulse aus der Stimulationsspannung, die mit der Frequenz der Impulse vom Impulsgenerator synchronisiert sind, einem Amplitudenregler zur Einstellung der Intensität der stimulierenden Impulse, und einer Versorgungseinheit für die Versorgung der genannten Einheiten mit Betriebsspannung besteht, wobei der Ausgang des Verzögerungsgliedes zum Takteingang des Transverters und des Impulsgenerators, der Ausgang vom Transverter zur Elektrode der einen Polarität, und der Ausgang vom Amplitudenregler zur Elektrode der anderen Polarität geführt sind und der Ausgang des Impulsgenerators an den Steuereingang des Choppers und die Eingänge des Amplitudenreglers an den Ausgang des Transverters und das ungeerdete Ende des gesteuerten Kanals des Choppers angeschlossen sind.

Bei der erfindungsgemäßen Schaltungsanordnung kann das von außen getriggerte Verzögerungsglied vorteilhaft durch ein von innen getriggertes, frei laufendes Verzögerungsglied ersetzt werden.

Bei einer Weiterentwicklung der erfindungsgemäßen Schaltungsanordnung können durch Vervielfachung der Komponenten und gegebenenfalls Einfügen von zusätzlichen Verzögerungsgliedern, Phasenspaltern und Phasenverschiebern mehrere Kanäle ausgebildet werden.

Bei einer besonders vorteilhaften Ausführungsform der erfindungsgemäßen Schaltungsanordnung kann der Impulsgenerator mit einem Modulator zur Erzeugung vom Impulsen mit einer monoton steigenen Impulsbreite in jeder Stimulationsperiode ergänzt werden.

Im folgenden wird die Erfindung anhand der - in der Zeichnung beispielsweise dargestellten - Ausführungsformen näher erläutert wobei

Fig. 1 das Blockschaltbild der erfindungsgemäßen Schaltungsanordnung,

Fig. 2 die Schaltung von einem Teil vom Blockschaltbild nach Fig. 1 mit einem von außen getriggerten Verzögerungsglied,

Fig. 3 die Schaltung von einem Teil vom Blockschaltbild nach Fig. 1 mit einem Verzögerungsglied, das sowohl von außen triggerbar ist, als auch frei laufend funktioniert,

Fig. 4 die Schaltung vom Impulsgenerator mit einem Impulsbreitenmodulator ergänzt und

Fig. 5 die vollständige Schaltung einer Ausführung der erfindungsgemäßen Schaltungsanordnung zeigt.

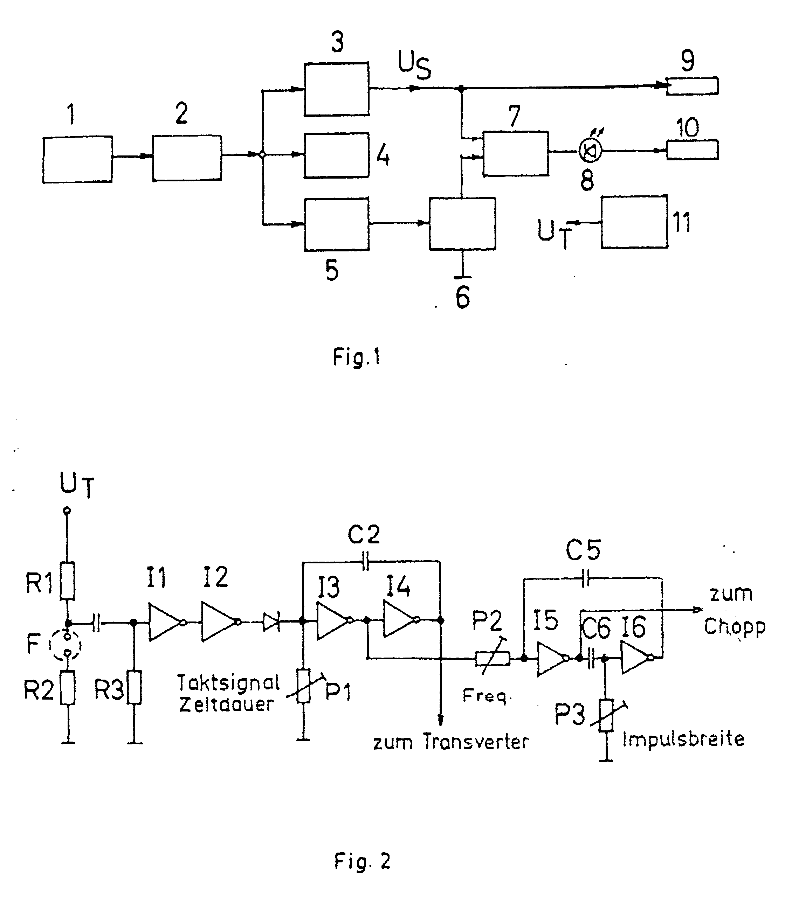

Der Aufbau der erfindungsgemäßen Schaltungsanordnung wird anhand der Fig. 1 erläutert. Diese Ausführungsform besteht aus einem Sensor 1, einem Verzögerungsglied 2, einem Transverter 3, einem Modulator 4, einem Impulsgenerator 5, einem Chopper 6, einem Amplitudenregler 7, einem Anzeigeelement 8, Elektroden 9 und 10 mit unterschiedlicher Polarität und einer Versorgungseinheit 11. Im Sensor 1 ist ein Schalter F für die Wahrnehmung der Betätigung vom Patienten oder von einer anderen Person. Das elektrische Signal vom Sensor 1 wird von einem Startsignalformer zu einem entsprechenden rechteckförmigen Startimpuls geformt. In Fig. 2 sind zum Beispiel für die Ausführung dieser Funktion Inverter I1 und I2 vorgesehen. Diese Inverter sorgen weiterhin auch dafür, daß ein Prellen vom als Schalter ausgebildeten Fühlerelement oder ein unbeabsichtigter Neustart die Funktion der Schaltung nicht stört. Der Startimpuls veranlaßt das hier aus den Invertern 13 und 14 als monostabiler Multivibrator ausgebildete Verzögerungsglied 2 zum Erzeugen eines Rechtecksignals mit einer voreinstellbaren Impulsdauer. Die Impulsdauer dieses Rechtecksignals kann mit Hilfe des Potentiometers P1 eingestellt werden. Der Ausgang des Verzögerungsgliedes 2 ist dem Eingang des Modulators 4, sowie dem Takteingang vom Transverter 3 und vom Impulsgenerator 5 zugeführt. Während des rechteckförmigen Taktimpulses vom Verzögerungsglied (d. h. während der Stimulierung) erzeugt der Transverter 3, der vorzugsweise aus einem LC-Oszillator und einem Gleichrichter zusammengestellt ist, aus der Betriebsspannung $U_T$ eine Stimulationsspannung $U_S$ in der Größenordnung von etwa 100 V. Dadurch, daß der Oszillator nur während der Stimulierung funktioniert, wird eine beachtenswerte Energieeinsparung erzielt, weil die Stromaufnahme in den Impulspausen nur einige nA beträgt. Dadurch wird bei Batteriebetrieb eine wesentlich längere Funktion gesichert.

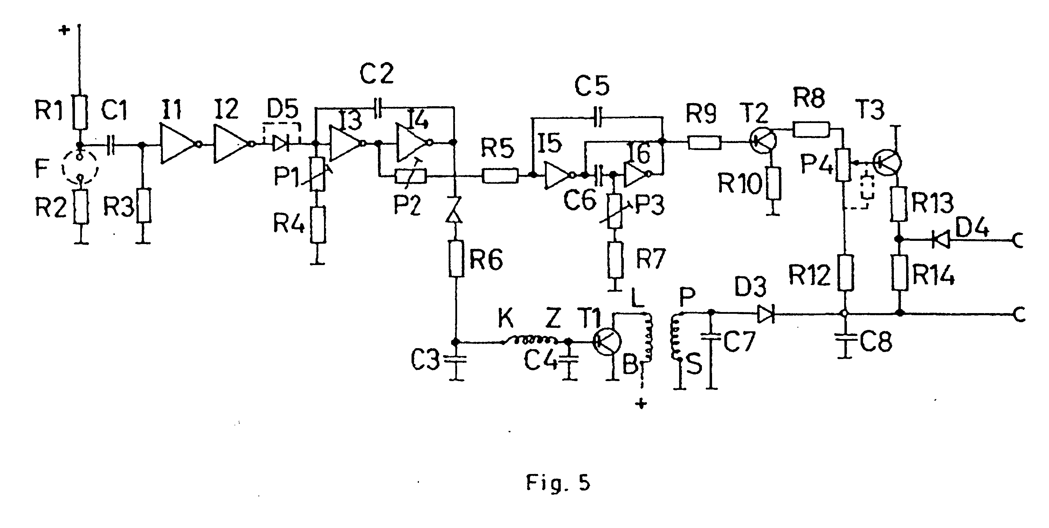

Die genannte Stimulationsspannung $U_S$ ist direkt an die Elektrode 9 mit positiver Polarität angeschlossen. Der negativen Elektrode 10 werden die Impulse vom Chopper 6, d. h. vom Impulsgenerator 5 abgenommene und vom Schalttransistor T2 verstärkte Stimulationsimpulse zugeführt. Die Spitzenspannung kann mittels Potentiometer P4 an der Basis vom Transistor T3 zur Amplitudenregelung (siehe Fig. 5) voreingestellt werden. Der Steuereingang vom Chopper 6 (also der Basis vom Transistor T2) ist mit dem Ausgang des getakteten Impulsgenerators verbunden, d. h. die Perioden der Unterbrechung werden durch die Frequenz und Impulsbreite der Impulsreihen (bursts) vom Impulsgenerator 5 bestimmt. Diese beiden Parameter können mit Hilfe der Potentiometer P2 und P3, die zum Impulsgenerator gehören, eingestellt werden.

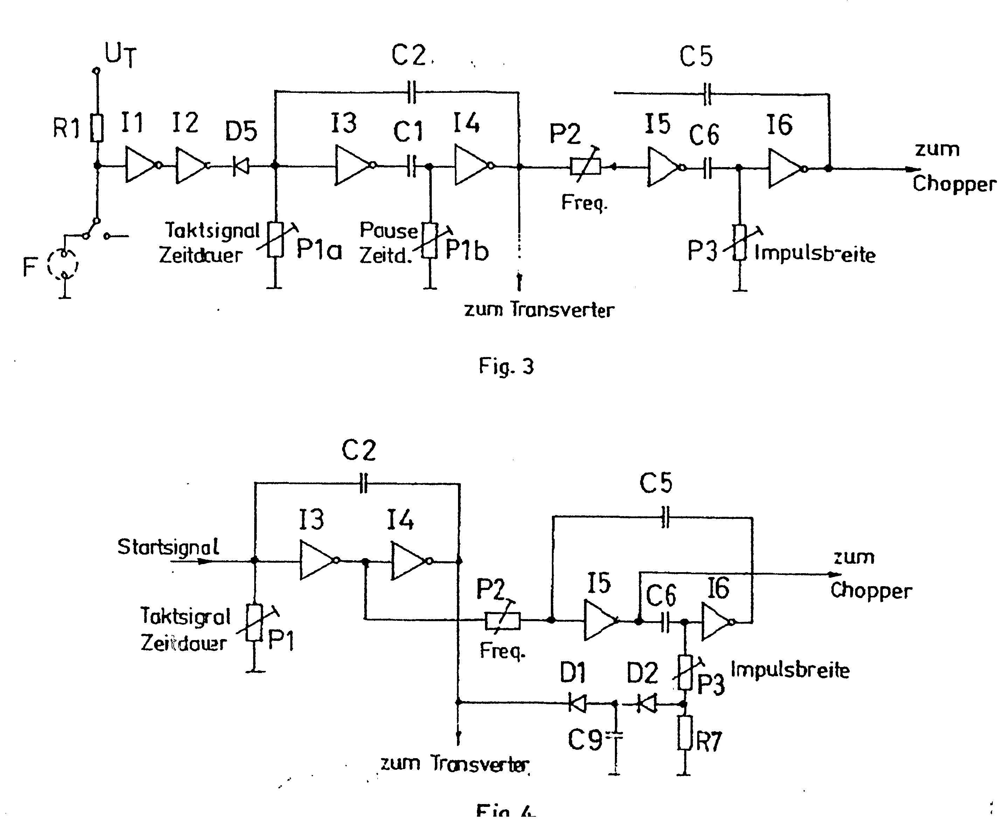

Der mit den Invertern 15 und 16 aufgebaute Impulsgenerator 5 ist eigentlich ein getakteter, astabiler (frei laufender) Mulitvibrator, der ebenfalls nur während der Stimulierung funktioniert, weil der Takteingang vom Impulsgenerator mit einem Ausgang A vom Verzögerungsglied 2 verbunden ist. In Fig. 2 und Fig. 5 hat das Verzögerungsglied 2 zwei Ausgänge A und B, deren Taktimpulse mit entgegengesetzter Phase den Takteingängen vom Transverter und vom Impulsgenerator zugeführt sind. Es ist aber, wie in Fig 3 ersichtlich, auch möglich, daß nur der eine Ausgang verwendet wird. Aus diesem Grund ist im Blockschaltbild in Fig. 1 wegen der besseren Übersicht nur ein Ausgang dargestellt.

Die bisher beschriebene Schaltung erfüllt schon die Zielstellung der Erfindung, aber eine weitere günstige Variante ergibt sich, wenn zwischen dem Ausgang B vom Verzögerungsglied 2 und den Modulationseingang vom Impulsgenerator 5 ein Modulator 4 eingefügt wird. Der Aufbau des Modulators 4 ist in Fig. 4 dargestellt. Der eingebaute Kondensator C9 führt eine Impulsbreitenmodulation dadurch aus, daß bei jedem einzelnen Impuls über die Diode D2 auf eine höhere Spannung geladen wird. Dadurch wrd der Widerstand R7 immer weniger geshuntet, also die nacheinander erzeugten Impulse weisen eine stetig wachsende Impulsbreite auf. Am Ende der Impulsreihe wird der Kondensator C9 über die Diode D1 entladen und auf diese Weise wiederholt sich der ganze Vorgang bei einem neuen Starten. Die Impulsbreitenmodulation diese Art erhöht die Belastung der Muskeln kontinuierlich, wodurch die unangenehme Stromempfindung und eine plötzliche Belastung der Muskeln vermieden wird.

In bestimmten Fällen kann es zweckmäßig sein, daß die erfindungsgemäße Schaltungsanordnung nicht nur von außen sondern auch von innen getriggert, d. h. frei laufend, in einer sich periodisch wiederholenden Betriebsart funktioniert. In Fig. 3 ist eine Schaltung mit einem Verzögerungsglied dargestellt, die in beiden der oben beschriebenen Betriebsarten funktioniert. Die hierbei verwendeten Inverter 13 und 14 sind zu einem synchronisierbaren astabilen Multivibrator mit hoher Zeitkonstant zusammengestellt. Bei dieser Lösung ist sowohl die Taktzeit oder Impulszeit (Dauer der Stimulation) als auch die Pausenzeit einstellbar.

Bisher wurde eine Schaltungsanordnung zur Stimulierung eines Muskels oder einer Muskelgruppe beschrieben. Die erfindungsgemäße Schaltungsanordnung ermöglicht aber auch eine Erweiterung der stimulierenden Leitungen (Kanäle). Durch Vervielfachung der Kompenente können gleichzeitung mehrere Muskelgruppen stimuliert werden. Durch Einfügen von zusätzlichen Verzögerungsgliedern, Phasenspaltern und Phasenverschiebern sind diese Kanäle zur zeitlich verschobenen Stimulierung der einzelnen Muskelgruppen fähig.

Die Ursache einer Lähmung oder teilweisen Lähmung ist oft eine Störung der Nervenverbindungen (Verletzung der Nerven, die zu den Muskeln führen oder des Rückenmarks in Querrichtung). In solchen Fällen wird der entsprechende Abschnitt des Rückenmarks freigelegt und es werden dann die Nervenwurzeln unter der harten Rückenmarkhaut gesucht, die für eine spezifische Muskelreaktion (Urinieren durch Zusammenziehen der Blase, Stuhlabgabe durch Zusammenziehen der Muskulator vom Anus, usw.) verantwortlich sind. Auf die Nervenwurzeln werden dann spezielle Elektroden aufgesetzt, die über eine Leitung mit einem Empfänger verbunden sind. Dieser Empfänger ist unter die Haut eingebaut. Auf die Hautoberfläche wird ein Sender aufgesetzt, der mit dem Stimuliergerät verbunden ist. Beim Drücken des Schalters am Reizstromgerät wird der Impulsgenerator aktiviert und eine Impulsreihe zum Sender geleitet. Die Impulsreihe wird erst beim Loslassen des Schalters unterbrochen. Der Empfänger unter der Haut nimmt die Signale des Senders auf und setzt diese in elektrische Spannungsimpulse um, die zu den Elektroden und damit zu den Nervenwurzeln geleitet werden. Der Effekt dieser Funktion ist z. B. das Urinieren, Stuhlabgabe, usw.. Falls sowohl die Nervenwurzeln für das Urinieren als auch die für die Stuhlabgabe mit Elektroden versehen werden, können am Stimuliergerät zwei Schalter (Druckknöpfe) angebracht und die Sekretion mit diesen auf einmal oder zeitlich getrennt gesteuert werden.

Bei einer anderen Anwendung kann die erfindungsgemäße Schaltungsanordnung bei der Behändlung von Behbehinderten, z. B. Patienten mit Peroneus Paresis nützlich sein. Die Patienten mit dieser Diagnose sind nicht imstand, den Fuß vom Erdboden zu heben, so daß ein Gehen sehr erschwert ist. Diese Patienten helfen sich deshalb entweder durch Zirkumdukation der unteren Extremitäten oder durch Heben der Hüften, das durch Veränderung der statischen Belastung schwere Nachfolgen haben kann. Bei solchen Patienten können Elektroden auf der Hautoberfläche und ein Schalter im Schuhabsatz verwendet werden. Wenn der Patient mit einer solchen Vorrichtung den defekten Fuß hebt, vermindert sich der Druck auf dem Schalter im Schuhabsatz. Durch Betätigung des Schalters erzeugt das Stimuliergerät Reizstromimpulse mit entsprechender Frequenz und Amplitude. Die Elektroden sind in diesem Fall auf die Hautoberfläche über den zu stimulierenden Muskeln im Bereich des Peroneus Communis aufgesetzt. Durch die zugeführten Reizstromimpulse werden die Muskeln zusammengezogen und der Fuß gehoben (dorsal flectiert). Beim Gehen wird auf diese Weise der Fuß entsprechend der normalen Funktion bewegt. Durch Veränderung der Frequenz und der Amplitude der stimulierenden Impulse kann die Stromempfindung unter die Empfindungsschwelle gesenkt werden, so daß der gewünschte Effekt noch zustandekommt. Außerdem kann auch die Dauer der Stimulierung entsprechend der Schritthäufigkeit eingestellt werden, so daß der Fuß weder zu früh noch zu spät, sondern genau beim Aufsetzen abfällt. Die Funktion der Vorrichtung wird bei dieser Anwendung durch die Körperfunktion, in diesem Fall durch das Gehen gesteuert. Bei richtiger Einstellung wird eine normale Muskelfunktion ausgelöst und dadurch die Beschädigung korrigiert.

Durch Anwendung der erfindungsgemäßen Schaltungsanordnung wird die Mobilisierung der Patienten erleichtert, die Dauer der Behandlung und der Heilung und damit die Inanspruchnahme des Krankenhauses verkürzt. Die Schaltung sichert eine den Patienten angepaßte Behandlung auch ohne eine Behandlungsperson oder Aufsicht eines Arztes.

## Liste der verwendeten Bezugszeichen

1 Sensor
2 Verzögerungsglied
3 Transverter
4 Modulator
5 Impulsgenerator
6 Chopper
7 Amplitudenregler
8 Anzeigeelement
9 Elektrode
10 Elektrode
11 Versorgungseinheit
A,B Ausgänge
C1-C9 Kondensatoren
D1-D3 Dioden
D4 Leuchtdiode
I1-16 Inverter
F Schalter
P1-P4 Potentiometer
T1-T3 Transistor
R1-R14 Widerstand
$U_S$ Stimulierungsspannung
$U_T$ Betriebsspannung

## Ansprüche

1. Schaltungsanordnung zur funktionsgemäßen, elektrischen Stimulierung gelähmter Muskeln oder Muskelgruppen mit einem Sensor, der vom Patienten oder einer anderen Person betätigt wird, mit Reizstromquelle und Elektroden zur Übertragung der Reizimpulse,
**dadurch gekennzeichnet,** daß die Reizstromquelle ein von außen getriggerter Burst-Generator ist, der aus einem vom Sensor (1) gesteuerten Verzögerungsglied (2) zur Bestimmung der Dauer der Stimulation, einem taktbaren Transverter (3) zur Erzeugung von Stimulationsspannung ($U_S$), durch Vervielfachung der Versorgungsspannung ($U_T$), einem taktbaren Impulsgenerator (5) zur Einstellung der Frequenz und Impulsbreite der stimulierenden Impulse, einem Chopper (6) zur Erzeugung der stimulierenden Impulse aus der Stimulationsspannung ($U_S$), die mit der Frequenz der Impulse von Impulsgenerator (5) synchronisiert sind, einem Amplitudenregler (7) zur Einstellung der Intensität der stimulierenden Impulse, und einer Versorgungseinheit (11) für die Versorgung der genannten Einheiten mit Betriebsspannung ($U_T$) besteht, wobei der Ausgang des Verzögerungsgliedes (2) zum Takteingang des Transverters (3) und des Impulsgenerators (5), der Ausgang vom Transverter (3) zur Elektrode (9) der einen Polarität, und der Ausgang vom Amplitudenregler (7) zur Elektrode (10) der anderen Polarität geführt sind und der Ausgang des Impulsgenerators (5) an den Steuereingang des Choppers (6) und die Eingänge des Amplitudenreglers (7) an den Ausgang des Transverters (3) und das ungeerdete Ende des gesteuerten Kanals des Choppers (6) angeschlossen sind.

2. Schaltungsanordnung nach Anspruch 1, dadurch gekennzeichnet, daß der Impulsgenerator (5) einen Modulationseingang aufweist, an den ein Modulator (4) angeschlossen ist.

3. Schaltungsanordnung nach Anspruch 2, dadurch gekennzeichnet, daß der Modulator (4) eine monoton steigende Impulsbreite während jeder Stimulationsperiode erzeugt.

4. Schaltungsanordnung nach einem der Ansprüche 1 bis 3, dadurch gekennzeichnet, daß das Verzögerungsglied (2) von außen triggerbar ist.

5. Schaltungsanordnung nach Anspruch 4, dadurch gekennzeichnet, daß das Verzögerungsglied (2) ein monostabiler Multivibrator ist.

6. Schaltungsanordnung nach einem der Ansprüche 1 bis 3, dadurch gekennzeichnet, daß das Verzögerungsglied (2) von innen triggerbar ist.

7. Schaltungsanordnung nach Anspruch 6, dadurch gekennzeichnet, daß das Verzögerungsglied (2) ein astabiler Multivibrator ist.

8. Schaltungsanordnung nach einem der Ansprüche 1 bis 3, dadurch gekennzeichnet, daß das Verzögerngsglied (2) sowohl von außen als auch von innen triggerbar ist.

9. Schaltungsanordnung nach einem der Ansprüche 1 bis 3 oder 8, dadurch gekennzeichnet, daß das Verzögerungsglied (2) ein von außen synchronisierbarer astabiler Multivibrator ist.

10. Schaltungsanordnung nach einem der vorangehenden Ansprüche, dadurch gekennzeichnet, daß der Sensor (1) aus einem Fühlerelement und einem Startsignalformer besteht.

11. Schaltungsanordnung nach einem der vorangehenden Ansprüche, dadurch gekennzeichnet, daß das Fühlerelement aus einem Schalter ausgebildet ist.

12. Schaltungsanordnung nach einem der vorangehenden Ansprüche, dadurch gekennzeichnet, daß der Transverter (3) aus einem DC-Oszillator und einem Gleichrichter besteht.

13. Schaltungsanordnung nach einem der vorangehenden Ansprüche, dadurch gekennzeichnet, daß der Impulsgenerator (5) ein taktbarer astabiler Multivibrator ist.

14. Schaltungsanordnung nach einem der vorangehenden Ansprüche, dadurch gekennzeichnet, daß im Chopper (7) zur Unterbrechung der Stimulationsspannung ($U_S$) ein Schalttransistor dient.

15. Schaltungsanordnung nach einem der vorangehenden Ansprüche, dadurch gekennzeichnet, daß in die Leitung der einen Elektrode (9 oder 10) ein Anzeigeelement (8) zur Anzeige der Stimulationsspannung vorgesehen ist.

16. Schaltungsanordnung nach Anspruch 15, dadurch gekennzeichnet, daß das Anzeigeelement eine Leuchtdiode ist.

17. Schaltungsanordnung nach einem der vorangehenden Ansprüche, dadurch gekennzeichnet, daß der Amplitudenregler (7) aus einem Spannungsteiler und einem Emitterfolger besteht.

18. Schaltungsanordnung nach einem der vorangehenden Ansprüche, dadurch gekennzeichnet, daß durch Vervielfachung der Komponenten(1 bis 10) und gegebenenfalls Einfügen von zusätzlichen Verzögerungsgliedern, Phasenspaltern und Phasenverschiebern mehrere Kanäle ausgebildet sind.

Fig.1

Fig. 2

Fig. 3

Fig. 4

EP 0 348 535 A1

Fig. 5

EP 0 348 535 A1

Europäisches
Patentamt

**EUROPÄISCHER RECHERCHENBERICHT**

Nummer der Anmeldung

EP 88 10 8548

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | SZABADALMI KÖZLÖNY, Band 92, EVF, 1987, EV, 27. Februar 1987, Seiten 111-112; & HU T/40766 (KOCSI) <br> * Zusammenfassung * <br> ----- | 1-18 | A 61 N 1/36 |

**RECHERCHIERTE SACHGEBIETE (Int. Cl.4)**

A 61 N

Der vorliegende Recherchenbericht wurde für alle Patentansprüche erstellt

| Recherchenort | Abschlußdatum der Recherche | Prüfer |
|---|---|---|
| DEN HAAG | 27-01-1989 | LEMERCIER D.L.L. |